# EUROPEAN PATENT APPLICATION

(11) **EP 1 533 037 A1**
(43) Date of publication of application: **25.05.2005**
(21) Application number: 04257290.9
(22) Date of filing: 24.11.2004
(51) Int. Cl.: B01L 3/00, B09B 3/00, A61L 11/00

(54) **Method and device for sanitizing disposable biochips**

(30) Priority: 24.11.2003 US 722086
(71) Applicant: Hewlett-Packard Development Company, L.P., Houston, TX 77070 (US)
(72) Inventor: Chapman, Charles B., San Diego, CA 92025 (US)
(74) Representative: Tollett, Ian

(57) **Abstract**

Biochips (10) include a substrate (14), a region (16) of the substrate having components (18) being configured to interact with a biological substance, and a heating device (12) integrated with the substrate. The heating device can be configured to generate heat over substantially all of said region, wherein the heat produces a temperature sufficient to pyrolyze the biological substance. The bio-analysis system can include a biochip having a region configured for conducting a biological assay on a biological substance, and an integrated device (50) including an analysis chamber (52) and a pyrolysis chamber (54). The analysis chamber can be configured for receiving the biochip and performing a biological assay on the region within the analysis chamber. The pyrolysis chamber also can be configured for receiving the biochip, wherein upon applying heat to the region within the pyrolysis chamber, the biological substance is pyrolyzed.

## Description

The present invention relates to a disposable biochip and to a bio-analysis system. More particularly, the present invention involves disposable biochips, which are sanitized prior to disposal.

As the realm of biotechnology and bio-analytical systems expands, biomolecules are becoming increasingly used for many analytical purposes. Generally, biomolecules are used in assays for determining the presence of chemical and/or biological substances, which can include determining whether a biological sample contains a particular molecular structure of interest. Also, assays employing biomolecules can be used to identify compounds within a biological sample including nucleic acids, proteins, peptides, polypeptides, toxins, pharmaceuticals, carcinogens, poisons, allergens, and infectious agents, to name a few. Accordingly, when these assays utilize bio-analytical systems, they can be used for the diagnosis and/or treatment of disease, to develop new compounds for pharmaceutical, medical or industrial purposes, or for detecting chemical and/or biological substances in various settings.

These assays and bio-analytical systems typically use bio-analysis devices employing biochips configured for certain experiments and/or detection studies. As a consequence of performing assays on biochips, the biochip can become contaminated with a biological substance. Biohazardous materials generally require special procedures for handling, decontamination, and/or disposal. Thus, the sanitization of a contaminated biochip may be required prior to disposal.

When bio-analytical studies are done in a laboratory setting, expensive corresponding infrastructure is typically required to handle biohazardous waste. Thus, the laboratory may be required to install autoclaves, incinerators, and other means to dispose of such waste, or at least properly decontaminate such waste prior to disposal. Another option for such laboratories may be to contract with special biowaste services or companies set up for the disposal of biohazardous waste.

Currently, as microcircuitry enables computer driven devices to become smaller, bio-analytical systems are becoming portable, or even handheld, e.g., laptops and PDAs. Correspondingly, the opportunity for on-location bio-analytical assays may become more prevalent, e.g., handheld pathogen detectors. As a consequence, when portable and/or handheld bio-analytical systems are more widespread, there will be a large demand for consumables to be used with these systems. Thus, disposable biochips for use in bio-analytical studies may also become more desirable, accessible, and/or affordable. However, after use with biological substances, these disposable biochips may constitute biohazardous waste, thereby increasing the difficulty for disposal.

A number of problems may arise with the special requirements to dispose of biohazardous wastes, such as with contaminated disposable biochips. Accordingly, an operator of such bio-analytical assays may be required to conduct the assays within a laboratory setting equipped with the aforementioned means for decontamination and/or disposal. Alternately, when the operator performs a bio-analytical assay on-location or outside of the laboratory, they may be required to transport the potentially biohazardous biochip back to the laboratory or another facility setup for proper decontamination and/or disposal thereof. Thus, there is a need for methods and/or devices to render disposable biochips harmless by decontaminating the biochip more conveniently.

It has been recognized that it would be advantageous to provide methods and devices to sanitize disposable biochips, and/or chemically change a biological substance thereon. In one embodiment of the present invention, a disposable biochip can include a substrate having a region configured for interacting with a biological substance, and further including a heating device. In addition, the heating device can be integrated with the substrate, and configured to generate heat over substantially all of the region. The heat can produce a temperature sufficient to pyrolyze the biological substance.

In another embodiment, a method for testing and pyrolyzing a biological substance can use a disposable biochip. The method can include conducting an assay of a biological substance on a disposable biochip. In addition, the biochip can include an integrated heating device, which can be configured for generating heat such that the biological substance is brought to a temperature sufficient to pyrolyze the biological substance.

In another embodiment, a bio-analysis system can include a biochip and an integrated analysis/pyrolysis device. The biochip can have a region configured for conducting biological assays. Also, the integrated device can have an analysis chamber that is configured for receiving the biochip, and can also be configured for performing a biological assay on the region of the biochip. Additionally, the integrated device can have a pyrolysis chamber that is configured for receiving the biochip, and also can be configured for containing the biochip when heat is applied thereto, where the heat can be applied to the region to raise the temperature to pyrolyze a biological substance.

Alternatively, a method for testing and pyrolyzing a biological substance using a bio-analysis system can comprise multiple steps. Such steps include contacting a region of a disposable biochip with a biological substance, providing an integrated bio-analysis device having an analysis chamber and a pyrolysis chamber, and introducing the biochip into the analysis chamber. Once in the analysis chamber, the step of conducting an assay on the region within the analysis chamber can occur, as well as transferring the biochip from the analysis chamber to the pyrolysis chamber. The step of generating heat in the pyrolysis chamber such that the biological substance is brought to a temperature sufficient to pyrolyze the biological substance can also be carried out.

In another embodiment, a bio-analysis system can comprise a biochip and a housing having an opening configured for receiving the biochip. The biochip can have a region configured for conducting a biological assay on a biological substance. An analysis chamber can also be present that is configured for receiving the biochip from the opening, wherein the analysis chamber is further configured to perform a biological assay on the region. A pyrolysis chamber can also be present that is configured for receiving the biochip from the analysis chamber, wherein upon applying heat to the region within the pyrolysis chamber, the biological substance is pyrolyzed, thereby decontaminating the region such that the biochip is substantially free of contaminants.

Additional features and advantages of the invention will be apparent from the detailed description which follows, taken in conjunction with the accompanying drawings, which together illustrate, by way of example, a number of preferred embodiments of the invention.
FIG. 1 illustrates a schematic view of an embodiment of a biochip in accordance with the present invention;
FIG. 2 illustrates a schematic view of another embodiment of a biochip in accordance with the present invention; and
FIG. 3 illustrates a schematic view of an integrated device in accordance with embodiments of the present invention.

Reference will now be made to the figures and exemplary embodiments, and specific language will be used herein to describe the same. It will nevertheless be understood that no limitation of the scope of the invention is thereby intended. Alterations and further modifications of the inventive features described herein, and additional applications of the principles of the invention as described herein, which would occur to one skilled in the relevant art and having possession of this disclosure, are to be considered within the scope of the invention. Further, before the figures and particular embodiments of the present invention are disclosed and described, it is to be understood that this invention is not limited to the particular process and materials disclosed herein as such may vary to some degree. It is also to be understood that the terminology used herein is used for the purpose of describing particular embodiments only and is not intended to be limiting, as the scope of the present invention will be defined only by the appended claims and equivalents thereof.

In describing and claiming the present invention, the following terminology will be used.

It should be noted that, as used in this specification and the appended claims, the singular forms "a," "an," and "the" include plural referents unless the context clearly dictates otherwise.

As used herein, "disposable" refers to the design and construction of an object such that it is relatively inexpensive, thereby making it cost effective to be discarded after a single use, or after it is rendered unusable. For example, a disposable biochip would be such that reconditioning the biochip for further use can be cost prohibitive compared to discarding the biochip and using a new disposable biochip for further assays.

As used herein, "biochip" refers to a substrate that is. usable in assays with biological substances. Typically, a biochip can comprise nylon, silicon, ceramic, and/or other similar materials. Accordingly, the biochip can be used to detect molecules in biological mediums, or detect biological molecules in the environment or other various locations. For example, a biochip can be used with genomics, proteomics, DNA, oligonucleotides, RNA, SNPs, STRs, amino acids, peptides, polypeptides, proteins, antibodies, antigens, and aptamers. Also, a biochip can be used for association studies, biotinylation, DNA arrays, protein arrays, cell arrays, combinatorial chemistry, molecular imprinting, drug discovery, SNP arrays, 2D-PAGE, capillary electrophoresis, arrayed primer extension technology, comparative genomic hybridization, ELISA, RIA, FISH, in-situ hybridization, locked nucleic acids, MAGE antigens, micro-electrode arrays, micro-electro mechanical systems, PCR, RT-PCR, sequencing, double strand sequencing, radio frequency identification, SAGE, SELDI, TSA, and photolithography.

As used herein, "biological substance" refers to any aspect of a biological entity, substance arising from a living entity, or molecular construct with biological activity. As such, blood, skin, tissue, peritoneal fluid, CNS fluid, hair, fat, muscle, cells, or any aspect thereof are considered macroscopic examples of biological substances. On the other hand without limitation, DNA, oligonucleotides, RNA, SNPs, STRs, amino acids, peptides, polypeptides, antibodies, antigens, aptamers, proteins, enzymes, or lipids are considered microscopic examples of biological substances. In addition, bioactive agents, such as pharmaceuticals, that interact with various physiological constructs such as enzymes and receptor proteins can be referred to as biological substances. Further, any molecule or organism with bioactivity can also be classified hereunder. However, these examples are not to be limiting and the use of the term as described herein can also be applied to any molecule or organism of biological interest, which can arise from any biological entity or interact with a biological entity.

As used herein, "reagent" refers to any biological or chemical substance used in a biological assay to detect, measure, examine, or produce other substances, for example.

As used herein, "pyrolyze," "pyrolysis," or "pyrolyzed" refers to a heat induced chemical alteration of a substance. Accordingly, any decomposition or transformation of any compound caused by heat to render the compound harmless, or to change its configuration, can also be referred to as pyrolysis. Irreversible pyrolysis occurs when the decomposition or chemical transformation of a chemical or biological substance cannot be reversed to the original state or formation without undergoing multiple steps. On the other hand, reversible pyrolysis occurs when the decomposition or chemical transformation of a chemical or biological substance can be reversed in a single step. Pyrolysis of a biological substance on a biochip can degrade the substance and render the substance altered enough for the biochip to be decontaminated or sanitized. Thus, either reversible or irreversible pyrolysis can decontaminate a biochip, where the biochip can then be disposed without further processing related to eliminating biohazardous waste. Alternatively, though a reason for pyrolyzing substances on a biochip that emphasized in the present disclosure is for decontamination purposes, an equally valid reason for pyrolyzing a biochip can be related to privacy issues. For example, if a biochip was prepared for analyzing a biological substance that was collected from a human source, then the biological substance will likely contain DNA or other traceable molecular structures. Thus, pyrolyzing a substance on the biochip may be to destroy or denature the DNA such that it cannot be traced to the individual that supplied to sample for the analysis. Thus, when referring to pyrolysis throughout the present disclosure as it relates to decontamination, it is understood that this purpose can be replaced with the desire to protect the privacy of individuals as well. Additionally, it is understood that both decontamination goals as well privacy goals can both be met in accordance with embodiments of the present invention. It is also possible to decontaminate a biochip without removing the privacy information, and *vice versa*, depending on the pyrolysis conditions.

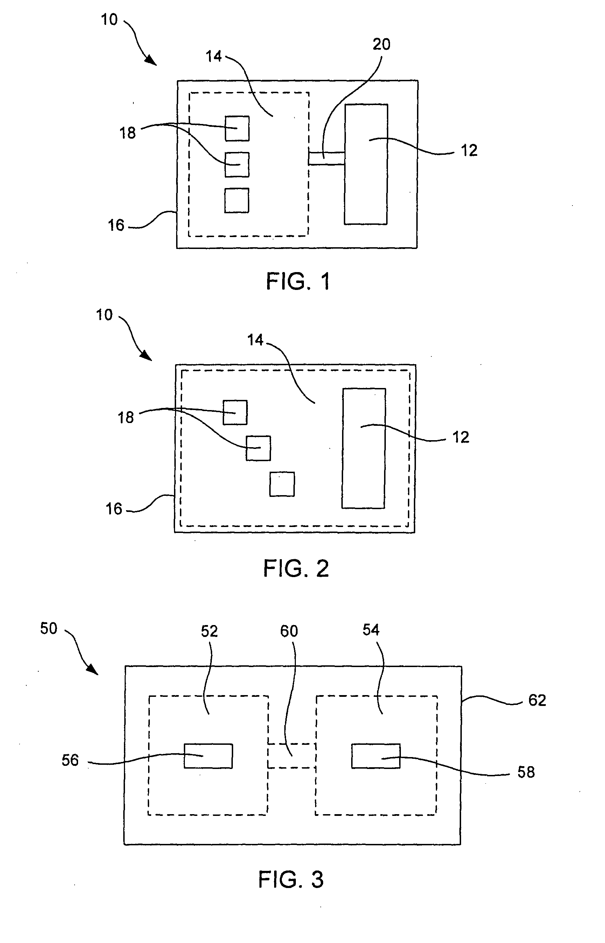

Referring now to FIGs. 1 and 2, two simplified schematic diagrams of biochips 10 in accordance with the present invention are shown. The biochip can include a heating device 12 integrated with a substrate 16. Such a biochip can have an assay region 14 on the substrate, where the assay region can be used for a variety of bio-analytical assays. Accordingly, the region can have components 18 that can be configured to interact with a biological substance, where the interactions can occur during an assay. The components can be mechanical, electrical, chemical, biological, structural, or the like, as long as the components are interactive, or potentially interactive, with a reagent being applied to the biochip. If the substance being tested for is not in the reagent, then the components may provide an alternative action, or may remain or be rendered inactive.

In different aspects, the assay region 14 can be of any size with respect to the substrate, and on either side, both sides, or throughout the substrate 16. As such, FIG. 1 shows the region to be on a portion of the substrate separate from the heating device 12. Alternately, FIG. 2 shows the assay region to be substantially present on an entire side of a substrate, wherein the assay region contains the heating device therein.

Also, the heating device 12 can be configured such that heat is generated over substantially all of the assay region 14. This can be accomplished with a thermocoupler 20 as in FIG. 1, or with the orientation of the heating device being within the region as in FIG. 2. Accordingly, the heating device can generate heat by an electrical heating element, infrared radiation, microwave radiation, redox chemical reactions, electromagnetic energy, or the like. In one embodiment, the heating device can be a resistive heater element.

When a biological substance is assayed on a biochip 10, the assay region 14 can become contaminated. In a macroscopic view, blood, tissue, and other biological substances can constitute biohazardous waste, where routine experimental protocols can require an experimenter to perform extraneous additional steps to ensure the biochip used is decontaminated. However, any biological substance can contaminate the biochip. Decontamination can be required under certain safety guidelines to ensure that potential infectious agents or other contaminates are rendered harmless. Alternatively, the pyrolysis of a biological substance can be desired for privacy purposes. The heating device 12 can be configured to generate heat to produce a temperature sufficient to pyrolyze a biological substance. In one embodiment, the heat can decontaminate the region such that the biochip is substantially free of contaminants, wherein the decontamination can be by way of pyrolyzing biological substances. Thus, when the temperature is substantially increased and a biological substance is pyrolyzed, the biochip may be considered to be sanitized. In either aspect, the decontamination or decomposition for privacy purposes can be accomplished with either irreversible pyrolysis or reversible pyrolysis, which can be achieved by an increase in temperature.

With the previously described figures in mind, the heating device 12 can be configured to generate heat over the assay region 14 in a manner that assists in conducting a bio-analytical assay. Thus, the assay occurs on a region of the biochip 10 having components 18 configured for conducting the assay. In this aspect of the invention, the heating device can be used to assist in conducting a bio-analytical study, and it can be configured to assist the components 18 in interacting with a biological substance. The heating device can also modulate the temperature of the biochip and/or the region through at least one heating cycle. A heating cycle can include increasing the temperature from Tₒ to T₂. The heating cycle can further include decreasing the temperature to either T₁ or back to T₀, or increasing the temperature to T₃. An example of such a heating cycle would be the process of increasing the temperature of a DNA molecule to induce the denaturing of the double strand, which causes the two strands to separate without pyrolyzing the DNA. Accordingly, the two strands of DNA can be further annealed back into a double stranded DNA by decreasing the temperature. Thus, the heating device can produce a temperature that is insufficient to pyrolyze the biological substance.
Additionally, the heating device can be configured to be overdriven upon command to produce a temperature sufficient to pyrolyze the biological substances typically after the assay or function is completed, as discussed previously.

In one aspect, the biochip 10 can be a disposable biochip. Many means for producing disposable biochips are currently available and well known to one of ordinary skill in the art. Additionally, the invention contemplates future advances in biotechnology and relates to developments in disposable biochips, configurations, and construction thereof. Thus, the disposable biochip is designed and produced in a manner such that disposal and the use of a new disposable biochip is cost beneficial when compared to replenishing the used disposable biochip.

With the previously described embodiments in mind, methods for testing and pyrolyzing a biological substance are also provided. Accordingly, in one aspect, a method for testing and pyrolyzing a biological substance can use a disposable biochip 10, and can include conducting an assay with a biological substance on the biochip. In addition, the method can include generating heat with an integrated heating device 12 such that the biological substance is brought to a temperature sufficient to pyrolyze the biological substance. In another aspect, a step of decontaminating the region such that the biochip is substantially free of contaminants can be included to sanitize the biochip. Alternatively, the biological substance can be pyrolyzed to protect privacy concerns.

In another aspect, prior to conducting an assay, the biological substance can be manually contacted to the region 14. Manually contacting the region can be performed by an experimenter through various means such as pipetting a solution containing the biological substance onto the region prior to the biochip being inserted into a bio-analytical device. Additional means for contacting a biological substance to the region can include, wiping, smearing, depositing, spraying, trapping, and/or various other modes of acquiring a biological substance onto the region. Another means for contacting the biological substance to the region may occur by placing the biochip in an environment having a biological substance, which can then self-deposit by the substance coming into contact with the region, e.g., via airborne transport.

Referring to FIG. 3, a simplified schematic diagram of an integrated device 50 in accordance with the present invention is provided. The integrated device can include a housing 62, an analysis chamber 52 within the housing, which can be configured for receiving a biochip, and a pyrolysis chamber 54 within the housing configured for receiving the biochip. Additionally, the integrated device can include a first opening 56 and a second opening 58 in accordance with embodiments of the invention. In one aspect, the first opening can be configured for receiving a biochip into the analysis chamber. In another aspect, the first opening can be configured for removing the biochip from the analysis chamber. In alternate aspects, the second opening can be configured for receiving the biochip into the pyrolysis chamber 54, and/or configured for removing the biochip from the pyrolysis chamber.

In another embodiment, the integrated device 50 can optionally include a transporter 60 configured for transferring the biochip from the analysis chamber 52 to the pyrolysis chamber 54. When the integrated device includes the transporter, the first opening 56 can be configured for receiving the biochip into the analysis chamber, and the second opening 58 can be configured for removing the biochip from the pyrolysis chamber. The transporter can transfer the biochip from the analysis chamber to the pyrolysis chamber. The transfer can occur by physically moving the biochip from one chamber to the other by a mechanical process, or dropping the biochip from the analysis chamber into the pyrolysis chamber when allowed by the orientation of the chambers within the integrated device. Examples of mechanical transporters include, without limitation, a lever, a rotating platform, a motor driven sled, a carriage on a track, a motor driven arm, a conveyor belt, or a platform that holds the biochip and moves the biochip from the analysis chamber to the pyrolysis chamber. Thus, in accordance with the present invention, the transporter can be in any configuration that can transfer the biochip from the analysis chamber to the pyrolysis chamber. In an alternative aspect, the integrated device can optionally exclude the second opening when the transporter is configured to transport the biochip between the analysis chamber and the pyrolysis chamber and the first opening is configured for receiving and removing or ejecting the biochip therefrom.

In accordance with the invention, the analysis chamber 52 can be further configured for performing a biological assay on a region on a biochip that has components configured for conducting assays when the region is within the analysis chamber. As such, the biochip can include a heating device as described previously, or can be another type of biochip configured for conducting biological assays therewith. The integrated device 50 can be designed to adequately provide the proper reagents to the analysis chamber for specific bio-analytical assays. Further, the analysis chamber can be designed to receive such reagents, and be configured for delivering the reagents to specific localities on the biochip. Additionally, the analysis chamber can be configured to perform a single type of bio-analysis assay, or can be configured to perform multiple assays. As such, the analysis chamber and/or the biochip can be equipped with various components to enable conducting biological assays. Some examples would include assays involving proteomics, genomics, gene mapping, DNA, RNA, PCR, RT-PCR, biological agent quantification, acquisition, and/or identification, and various other biologically related assays. The analysis chamber, and/or the biochip itself, can include components to enable analysis of assay conditions and results, which include light sensors, luminescence and chemiluminescence spectroscopy, mass spectroscopy, electrochemical potential sensors, quartz crystal microbalance sensors, and various imaging as well as analysis equipment. Alternately, such equipment can be associated with the analysis chamber without being within the analysis chamber.

In an aspect of the present invention, the pyrolysis chamber 54 can further be configured for containing the biochip during application of heat to the biochip. Accordingly, a heating device can generate the heat within the pyrolysis chamber. In accordance with FIGs. 1 and 2, as discussed previously, such a heating device can be onboard the biochip. Alternately, the heating device can be integrated within the device 50, and configured to affect contents within the pyrolysis chamber. In either case, the generation of heat to pyrolyze a biological substance and decontaminate a biochip, as discussed previously with respect to the heating device being integrated with the biochip 10, can further be applied to the heat generated within the pyrolysis chamber. The application of heat to the region in the pyrolysis chamber can increase the temperature to a level sufficient to reversibly or irreversibly pyrolyze a biological substance.

In another aspect, the first and second openings 56, and 58, respectively, can both be configured for receiving and/or removing the biochip with respect to the integrated device 50. Accordingly, the openings can be of any practical geometric shape or any depth. Also, the openings can include doors to substantially close the openings when engaged. Further, the openings can further include mechanisms to enable either receiving or ejecting a biochip therefrom.

In an additional aspect, the pyrolysis chamber 54 can be substantially thermally isolated from the analysis chamber 52. The thermal isolation can be by, insulation, distance, and other means to keep the heat generated in the pyrolysis chamber from substantially increasing the temperature of the analysis chamber. In some systems, it can be important to keep the heat from increasing the temperature of the analysis chamber because of the nature of the equipment disposed within or in association with the analysis chamber and utilized in bio-analytical assays. For example, such equipment can be thermosensitive, where excess heat can degrade or corrupt the functions thereof.

In another embodiment, the integrated device 50 can be portable, and can be designed for easy transfer to and from various locations. For example, the integrated device can be handheld, or can include a cart or carriage configured to carry the integrated device. Thus, the integrated device can be located in a laboratory and easily moved to an on-site location for conducting a bio-analytical assay as well as being capable of pyrolyzing a biological substance at the location. Making the device portable can include implementing the use of detachable carriages as well as integrated carriages designed to enable the portability of the integrated device.

Such an integrated device 50 can have an internal power supply such as a battery, and/or configured to be electrically coupled to an external power supply. In accordance with FIGs. 1 and 2, the biochip 10 can be configured to be electrically coupled to the bio-analysis device, and can utilize the power source that operates such a device. The use of power by the heating device can be during an assay, and/or to overdrive the heating device to increase the temperature to be sufficient to pyrolyze a biological substance. On the other hand, the electricity supplied by the bio-analytical device to the biochip does not necessarily have to overdrive the heating device, but can simply enable the heating device to achieve the requisite temperature for pyrolyzing the biological substance.

In another embodiment, a bio-analysis system can include a biochip, and an integrated device 50 having an analysis chamber 52 and a pyrolysis chamber 54. Additionally, the bio-analysis system can include a heating device. In one aspect, the heating device can be configured for generating heat in the analysis chamber to assist in a bio-analytical assay, as described previously. Also, the heating device can be within the pyrolysis chamber 54 by either being integrated with the biochip 10, or integrated with the pyrolysis chamber. Thus, the heating device can increase the temperature to a level sufficient to pyrolyze the biological substance, as described previously.

In another embodiment, the integrated device can be automated and configured for positively placing the biological substance on the assay region, thereby providing contact between the biological substance and the assay region while the biochip is within the device, but prior to introduction into the analysis chamber. Alternatively, the step of contacting the assay region with the biological substance can occur in the analysis chamber using automation.

It is to be understood that the above-referenced arrangements are illustrative of the application for the principles of the present invention. Numerous modifications and alternative arrangements can be devised without departing from the scope of the present invention while the present invention has been shown in the drawings and described above in connection with the exemplary embodiments(s) of the invention. It will be apparent to those of ordinary skill in the art that numerous modifications can be made without departing from the principles and concepts of the invention as set forth in the claims.

## Claims

1. A disposable biochip (10), comprising:
(a) a substrate (14);
(b) a region (16) of the substrate having components being configured to interact with a biological substance; and
(c) a heating device (12) integrated with the substance, said heating device being configured to generate heat over substantially all of said region, wherein the heat produces a temperature sufficient to pyrolyze the biological substance.

2. The biochip of claim 1, wherein the heating device is configured to be electronically coupled to a bio-analysis device.

3. The biochip of claim 1 or 2, wherein the heating device is within the region.

4. The biochip of claim 3, wherein the heating device within the region is further configured to assist the components in interacting with the biological substance.

5. The biochip of any preceding claim, wherein the heating device is configured to be overdriven upon command to produce the temperature sufficient to pyrolyze the biological substance.

6. A bio-analysis system, comprising:
(a) a biochip having a region configured for conducting a biological assay on a biological substance; and
(b) an integrated device (50) including an analysis chamber (52) and a pyrolysis chamber (54), said analysis chamber being configured for receiving the biochip and performing a biological assay on the region within the analysis chamber, said pyrolysis chamber also being configured for receiving the biochip, wherein upon applying heat to the region within the pyrolysis chamber, the biological substance is pyrolyzed.

7. The bio-analysis system of claim 6, further comprising a heating device, said heating device being configured to generate heat over substantially all of said region, wherein the heat produces a temperature sufficient to pyrolyze the biological substance.

8. The bio-analysis system of claim 6 or 7, wherein the integrated device is portable.

9. The bio-analysis system of any of claims 6 to 8, wherein the integrated device further includes a transporter configured for transferring the biochip from the analysis chamber to the pyrolysis chamber.

10. The bio-analysis system of any of claims 6 to 9, wherein the integrated device further includes a first opening and a second opening, said first opening being configured for receiving the disposable biochip into the analysis chamber, said second opening being configured for removing the biochip from the pyrolysis chamber.

11. The bio-analysis system of claim 7, wherein the biochip includes the heating device.

12. The bio-analysis system of claim 7, wherein the pyrolysis chamber includes the heating device.
